# EUROPEAN PATENT SPECIFICATION

(11) **EP 2 658 497 B1**
(45) Date of publication and mention of the grant of the patent: **04.01.2017**
(21) Application number: 11854449.3
(22) Date of filing: 16.11.2011
(51) Int. Cl.: A61F 13/53, A61F 13/493, A61F 13/49, A61F 13/84, A61F 13/42, A61L 15/56, B32B 37/12

(54) **ABSORBENT ARTICLE WITH STIMULATION COMPOSITE**
SAUGFÄHIGER ARTIKEL MIT EINEM STIMULATIONSVERBUNDSTOFF
ARTICLE ABSORBANT AVEC COMPOSITE DE STIMULATION

(30) Priority: 28.12.2010 US 930096
(43) Date of publication of application: 06.11.2013
(73) Proprietor: Kimberly-Clark Worldwide, Inc., Neenah, Wisconsin 54956 (US)
(72) Inventor: ZHOU, Peiguang, Appleton Wisconsin 54915 (US); CLOSE, Kenneth, B., New London Wisconsin 54961 (US); LONG, Andrew, M., Appleton Wisconsin 54915 (US); BREY, Gregg, M., Oshkosh Wisconsin 54904 (US); BENEDICT, Patsy, A., Omro Wisconsin 54963 (US)
(74) Representative: Davies, Christopher Robert
(86) International application number: PCT/IB2011/055134
(87) International publication number: WO 2012/090084

(56) References cited:
- WO-A1-2009/133745
- WO-A2-03/061541
- US-A- 5 702 376
- US-A1- 2002 169 427
- US-A1- 2005 096 623
- US-A1- 2005 228 349
- US-A1- 2006 142 715
- US-A1- 2006 142 716
- US-A1- 2007 083 172
- US-A1- 2007 083 172
- US-A1- 2009 157 022
- US-A1- 2011 152 816

## Description

### BACKGROUND

The present invention relates to absorbent articles that include a stimulation material. More specifically, the invention relates to an absorbent article, such as training pants, that provides the wearer with a noticeable sensation upon fluid contact.

Absorbent articles, for example, children's training pants, have been designed with a stimulation material such as temperature change particles to provide a temperature change sensation upon urination (a liquid insult). This is done in an attempt to enhance a child's recognition of when urination occurs. As can be appreciated, such recognition can be an important step in the toilet training process. For example, WO 2009/133745 discloses a disposable article having a temperature changing material mounted in a pocket. US 2011/0152816 discloses an absorbent article having a signal element including a stimulation material. US 2006/0142716 and US 2007/0081372 disclose articles with temperature change members.

Currently, stimulation material in a particulate form has been incorporated into a coform material and cut into pledgets for placement in absorbent articles. Stimulation material is trapped within the coform structure. Bonding between the coform fibers and the stimulation material is poor so that when the coform web is cut into pledgets, the stimulation material falls away from the pledget edges, resulting in a waste of the stimulation material of about 25 to about 30 percent.

In addition to the waste of stimulation material, the coform structure cannot hold more than about 300 gsm of the stimulation material without a significant loss of stimulation material due to poor bonding. This means that the sensation cannot be enhanced by increasing the amount of the stimulation material.

To increase sensation and overcome the problem of stimulation material waste due to the poor bonding, a stimulation laminate was developed in which the stimulation material was adhesively bonded to substrates in such a manner that the stimulation material covered a substrate completely. Several layers of stimulation materials were disposed between several layers of substrate. The laminate structure greatly improved the bonding efficiency of the stimulation materials, so that maximum add-on levels of these materials could be increased from 300 gsm to about 1000 gsm. More than 99% of stimulation materials were captured in the laminate structure during lamination process. There was virtually no loss or waste of stimulation material either during use or when pledgets were cut from the web of stimulation laminate.

Though the adhesive bonding of the stimulation material worked well to prevent loss of stimulation material in manufacturing and converting process conditions, it was found that the stimulation material migrated by being washed away from the part of the pledget area subjected to a direct liquid insult. The migration of stimulation materials due to a direct insult to the pledget lowers stimulation efficiency. Often the stimulation material is displaced to areas of the absorbent article that do not have contact with the wearer's skin. Because of this displacement, it is observed that less than about 50% stimulation composite is available to deliver some sensation to wearer. To add stimulation material to the laminate to compensate for migration would dramatically increases manufacturing costs.

Thus, there is a need for a pledget containing a stimulation material, wherein the stimulation material can provide an improved level of noticeable sensation (e.g. cooling, warming, tingling, and/or pressure). In addition, there is a need for a pledget containing a stimulation material, wherein the stimulation material is focused in a predetermined region with respect to a "target zone" at which a liquid insult occurs. Moreover, there is a need for a stimulation composite which exhibits a negligible waste of stimulation material when the stimulation composite is converted into pledgets. Overall, there is a need for a pledget having all the advantages of the laminate structure described above, while containing a reduced amount of stimulation material.

### SUMMARY OF THE INVENTION

From a first aspect, there is provided a method of making a stimulation composite as claimed in claim 1.

The terms upstream and downstream as used herein are relative to the machine direction. The method may comprise additional steps including the disposing of a body-facing substrate onto the stimulation blend, and applying pressure to the resulting stimulation composite.

Also disclosed herein is an absorbent article is made from several components, one of which is a stimulation composite that is disposed between a top sheet and an absorbent core. The stimulation composite is made from a stimulation blend that includes a stimulation material that is partially bonded to pressure- sensitive adhesive fibers. The stimulation blend and some pressure-sensitive adhesive fibers are arranged into a predetermined pattern and sandwiched between a body-facing substrate and a garment-facing substrate to form a stimulation composite. The stimulation composite is disposed between a body-facing substrate and a garment-facing substrate.

The absorbent article is further constructed from a backsheet, an absorbent core, and a topsheet. The absorbent core is located between the topsheet and the backsheet, and the stimulation composite is located between the absorbent core and the topsheet.

The stimulation composite may includes a stimulation blend made from a stimulation material that is mostly partially bonded to pressure-sensitive hot-melt adhesive fibers. The stimulation blend may be disposed between a body-facing substrate and a garment- facing substrate.

### BRIEF DESCRIPTION OF DRAWINGS

The patent or application file contains at least one drawing executed in color. Copies of this patent or patent application publication with color drawing(s) will be provided by the Office upon request and payment of the necessary fee.

While the specification concludes with claims particularly pointing out and distinctly claiming the subject matter that is regarded as the present invention, it is believed that the invention will be more fully understood from the following description taken in conjunction with the accompanying drawings. In the accompanying drawing figures, like reference numerals identify like elements, which may or may not be identical in the several exemplary embodiments that are depicted. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings are necessarily to scale.
Fig. 1 is a cross-sectional view of one embodiment of a stimulation composite of the present invention.
Fig. 2 is a plan view with a cut-away of the stimulation composite showing, in a schematic fashion, a stimulation blend according to one embodiment of the present invention.
Fig. 3 is a cross-sectional view of the embodiment shown in Fig. 2, taken at line 3-3.
Fig. 4 illustrates one embodiment of a process for constructing the stimulation composite of the present invention.
Figs. 5(A-F) are schematic plans of exemplary patterns of the stimulation blend according to the present invention.
Fig. 6 is a partial perspective view of one embodiment of a particle feeder of the present invention.
Fig. 6A is a perspective view of another embodiment of the particulate feeder having only one channel.
Fig. 7 is a perspective view of one embodiment of the particle feeder showing how panels can be used to separate the particle feeder and channel attachment.
Fig. 8 is a schematic plan view of one embodiment of the present invention depicting the manufacture of the stimulation composite having a pattern made by the stimulation blend.
Fig. 9 is a side view of one embodiment of a training pant fastened on one side and unfastened on the opposite side.
Fig. 10 representatively illustrates a cut-away plan view of the training pant of Figure 9 in an unfastened, stretched and laid flat condition, showing the body-side surface of the training pant and stimulation composite.

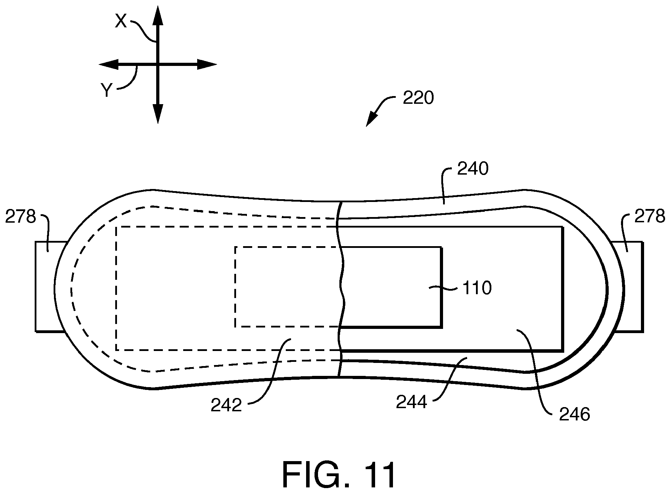

Fig. 11 is a feminine care pad having one embodiment of a stimulation composite of the present invention.
Fig. 12 is a perspective view of a thickness tester utilized in the Thickness Test.
Figs. 13-15 shows an array of thermal imaging photographs depicting cooling areas that develop after an insult is applied to a target zone of a pledget of stimulation composite.
Fig. 16 is a front perspective view of a test mannequin clothed with a training pant for a thermal imaging test.

### DEFINITIONS

It should be noted that, when employed in the present disclosure, the terms "comprises," "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

The term "absorbent article" generally refers to devices which can absorb and contain fluids. For example, personal care absorbent articles refer to devices which are placed against or near the skin to absorb and contain the various fluids discharged from the body.

The term "bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. Notwithstanding the foregoing, in the context of the present invention, all stimulation materials are bonded directly, without any intermediate elements, to the adhesives described herein.

The term "coform" refers to a blend of meltblown fibers and absorbent fibers such as cellulosic fibers that can be formed by air forming a meltblown polymer material while simultaneously blowing air-suspended fibers into the stream of meltblown fibers. The coform material may also include other materials, such as stimulation materials. The meltblown fibers and absorbent fibers are collected on a forming surface, such as provided by a foraminous belt. The forming surface may include a gas-pervious material that has been placed onto the forming surface. Exemplary coform processes are described in U.S. Patent Nos. 4,100,324 to Anderson et al.; 4,587,154 to Hotchkiss et al.; 4,604,313 to McFarland et al.; 4,655,757 to McFarland et al.; 4,724,114 to McFarland et al.; 4,100,324 to Anderson et al.; and U.K. Patent GB 2,151,272 to Minto et al.

The term "disposable" is used herein to describe absorbent articles that are not intended to be laundered or otherwise restored or reused as an absorbent article after a single use.

The terms "disposed on," "disposed along," "disposed with," or "disposed toward" and variations thereof are intended to mean that one element can be a separate structure bonded to or placed with or placed near another element.

The terms "elastic," "elasticized," "elasticity," and "elastomeric" and derivatives thereof mean that property of a material or composite by virtue of which it tends to recover its original size and shape after removal of a force causing a deformation. Suitably, an elastic material or composite can be elongated by at least 50 percent of its relaxed length and will recover, upon release of the applied force, at least 40 percent of its elongation.

The term "extensible" refers to a material or composite which is capable of extension or deformation without breaking, but does not substantially recover its original size and shape after removal of a force causing the extension or deformation (i.e., less than 40 percent recovery). Suitably, an extensible material or composite can be elongated by at least 50 percent of its relaxed length.

The term "fiber" refers to a continuous or discontinuous member having a high ratio of length to diameter or width. Thus, a fiber may be a filament, a thread, a strand, or any other member or combination of these members.

The term "health/medical absorbent articles" includes a variety of professional and consumer health-care products including, but not limited to, products for applying hot or cold therapy, medical absorbent garments, bed pads, and the like.

The term "hydrophilic" describes materials which are wetted by aqueous liquids in contact with the materials. The degree of wetting of the materials can, in turn, be described in terms of the contact angles and the surface tensions of the liquids and materials involved. Equipment and techniques suitable for measuring the wetability of particular fiber materials or blends of fiber materials can be provided by a Cahn SFA-222 Surface Force Analyzer System, or a substantially equivalent system. When measured with this system, materials having contact angles less than 90 degrees are designated "wettable" or hydrophilic, and fibers having contact angles greater than 90 degrees are designated "nonwettable" or "hydrophobic".

The term "insult surface" describes the body-facing surface that will make first contact with a fluid insult.

The term "meltblown fibers" refers to fibers formed by extruding a molten thermoplastic material through a plurality of fine, usually circular, die capillaries as molten threads, fibers or filaments into a high velocity, usually heated, gas (e.g., air) stream which attenuates the filaments of molten thermoplastic material to reduce their diameter. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super- Fine Thermoplastic Fibers" by K. D. Lawrence, R. T. Lukas and J. A. Young; and U.S. Patent No. 3,849,241 to Butin et al. and U.S. Patent No. 5,350,624 to Georger et al..

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblown processes, spunbond processes, air laying processes, wet laying processes and bonded-carded-web processes.

In the context of the present invention, the term "personal care absorbent articles" includes, but is not limited to, absorbent articles such as diapers, diaper pants, training pants, absorbent underpants, child care pants, and other disposable garments; feminine care products including menstrual pads, menstrual pants, panty liners, panty shields, interlabials; adult-care products including, pads such as breast pads, incontinence products, and urinary shields.

The term "pledget" in the context of the present invention refers to a portion cut from the web of stimulation composite 1 10 during a conversion process (either in-line or off-line the absorbent article production line).

The terms "spunbond" and "spunbonded fiber" refer to fibers which are formed by extruding filaments of molten thermoplastic material from a plurality of fine, usually circular, capillaries of a spinneret, and then rapidly reducing the diameter of the extruded filaments.

The term "target zone" is a pledget region to which a liquid insult is directed.

The term "% by weight," "weight %," "wt%" or derivative thereof, when used herein, is to be interpreted as based on the dry weight, unless otherwise specified.

The above mentioned terms may be defined with additional language in the remaining portions of the specification.

### DETAILED DESCRIPTION

In the present invention, the unique structure of the stimulation composite provides user stimulation after at least one insult. This is done by partially bonding the stimulation material to fibers of adhesive through a meltblown application to reduce or prevent loss of the stimulation material from the stimulation composite as it is converted into pledgets and as it is used by consumers. In addition, because the insult target zone can be determined, it is possible to place the stimulation material into the most desirable areas of the pledget. In some cases, it may be desirable to have stimulation material uniformly cover entire pledget surface closest to the body-facing surface, by covering the area outside the target zone. However, in most cases, it is desirable to have the stimulation material partially cover the pledget area closest to the body-facing surface.

Desirably, the partial coverage is located away from areas of the pledget which are directly subjected to insult to reduce or substantially eliminate waste of the stimulation material. Further, such a configuration can provide either the same or an improved sensation as can uniform coverage. Therefore, not only can the present invention positively affect the stimulation experience as it relates to a targeted zone, it can also reduce manufacturing/converting costs by using significantly less amounts of the expensive stimulation material.

The improved material of this invention (hereinafter referred to as "stimulation composite") is useful in personal care and health/medical disposable absorbent articles. An absorbent article of the present invention may have an absorbent core, and a topsheet and/or a backsheet. The absorbent core is disposed between the topsheet and the backsheet. The stimulation composite includes a garment-facing substrate to which a stimulation material and adhesive is attached thereto. In a most desired embodiment, the stimulation composite includes a body-facing, porous-substrate disposed on the body-facing surface of the stimulation blend.

### Stimulation composite

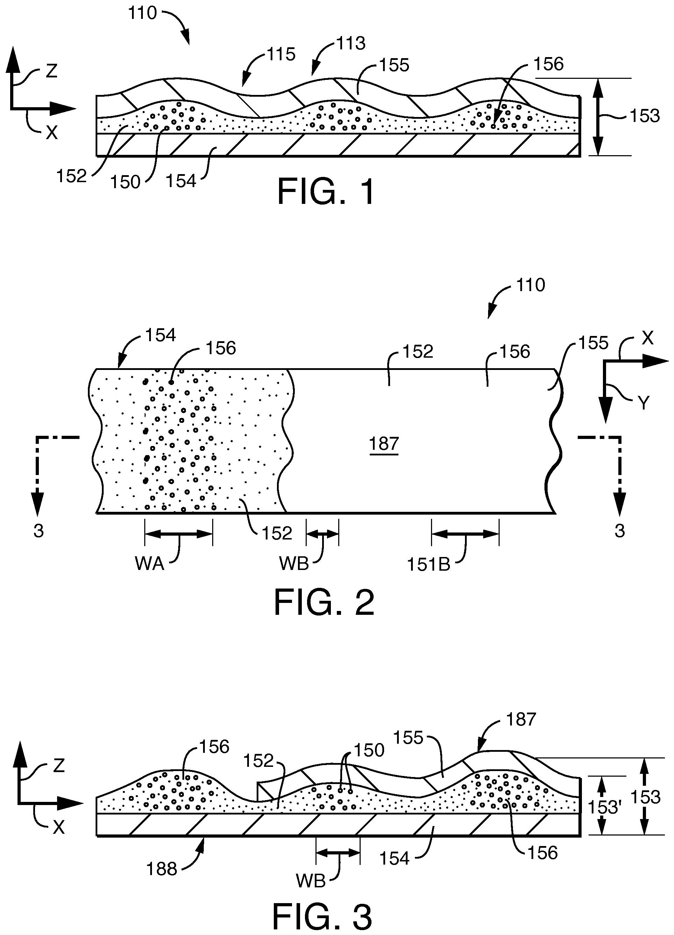

Referring to Fig. 1, in a desired embodiment, the stimulation composite 110 has four primary components: a stimulation material 150, an adhesive 152, and two substrates 154 and 155 (a body-facing substrate and a garment-facing substrate, respectively). The stimulation material 150 and adhesive 152 are comingled in a meltblown process to form a stimulation blend 156. The stimulation blend 156 and adhesive 152 are disposed between the two substrates 154, 155 to form the stimulation composite 110, (though it is recognized that it may not be necessary to include the substrate 154, a body-facing substrate). Each of these components are described in the following.

The purpose of stimulation material 150 is to provide the user with a perceptible sensation when a fluid insult is occurring and/or has occurred. The sensation may be a discernable temperature change, a tingling sensation, pressure or any sensation that can alert the wearer that a liquid insult has occurred.

The stimulation material 150 can be of any practical form, but it is desirably in the form of a solid. As it refers to stimulation materials, the term "solid" can include particles, flakes, fibers, agglomerates, granules, powders, spheres, pulverized materials or the like, as well as combinations thereof. The solids can have any desired shape such as, for example, cubic, rod-like, polyhedral, spherical or semi-spherical, rounded or semi-rounded, angular, irregular, and the like.

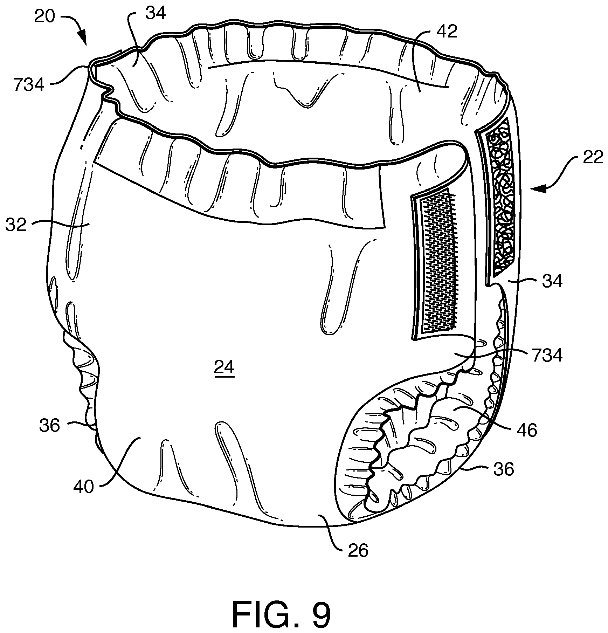

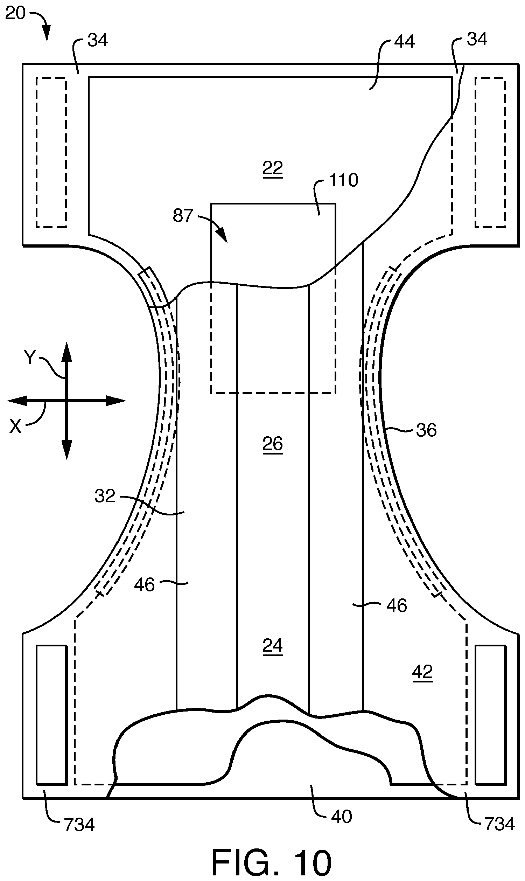

A disposable absorbent article 20 (shown in Figs. 9 and 10) may include a stimulation composite 110 formed into a pledget of a desired shape. The stimulation composite 110 has within it an adhesive 152 and stimulation material(s) 150 that dissolve in an aqueous liquid. These stimulation material(s) 150 are partially bonded to the adhesive 152. The solubility of the stimulation materials 150 is desirably from about 0.1 to about 6 grams of material per gram of water (g/g), such as from about 0.1 g/g to about 3 g/g.

As can be appreciated, the stimulation composite 110 of the present invention can be described as having a total amount of stimulation material 150, by weight. For example, in some aspects, the stimulation composite 110 can include about 0.5 to 20 grams of stimulation material 150, or about 1 to 15 grams of stimulation material, or about 1 to 10 grams of stimulation material.

In some aspects, one can define a particle size distribution of stimulation material 150 within the stimulation composite 110. It should be understood that in aspects where the stimulation material is in the form of particles, the invention can include particles having sizes ranging from less than about 90 microns (including nanoparticles) to greater than about 710 microns, as measured by the Particle Size Test.

In general, the stimulation material 150 is responsive to contact with an aqueous solution such as urine, menses or other aqueous body exudates to provide a stimulating effect, such as the absorption or release of heat, pressure, a tingling sensation and the like. In general, the mechanism by which this is accomplished is by dissolution of the stimulation material 150 in the aqueous solution, or by reaction of the material 150 in the aqueous solution. For example, the stimulation material 150 may include particles that have a substantial energy difference between a dissolved state and a crystalline state so that energy in the form of heat is absorbed or released to the environment upon contact with liquid. The selection of a particular stimulation material 150 and the determination of the amount to be used should be based in part on the desired stimulation effect.

In some aspects, the stimulation material 150 of the various aspects of the present invention can include a substance that provides a temperature change (referred to herein as a "temperature change agent") when contacted with an aqueous liquid and placed in indirect contact with the wearer's skin. "Indirect" because there may be one or more layers between the stimulation composite and the skin. In some aspects, the temperature change can be an absorption or release of heat that is noticeable to the wearer. Absorption of heat by the temperature change agent (also referred to herein as a "cooling agent") will provide the wearer with a cool sensation, while a release of heat by the temperature change agent (also referred to herein as a "warming agent") will provide the wearer with a warm sensation. Reference is made to U.S. Patent Application Publication No. 2004/0254549 to Olson, et al., for additional information regarding the mechanism by which the temperature change sensation is accomplished. The cooling agents or warming agents can be provided in particulate form for ease of processing in the described embodiments. To illustrate, in aspects where the stimulation material 150 is a temperature change agent, the stimulation composite 110 may suitably provide a temperature change (i.e., cooler, warmer or both) when insulted with an aqueous liquid of at least about 2 °C, such as at least about 5 °C, or at least about 10 °C, or between 3 °C and 15 °C.

In some aspects, the stimulation material 150 can include a temperature change agent in the form of a cooling agent, which can include those substances that absorb heat during dissolution from an insult. By way of example, polyols such as xylitol particles may be selected as cooling agents to provide a cooling sensation as xylitol particles absorb heat when dissolved in an aqueous liquid. Alternatively, other polyols such as sorbitol or erythritol may be advantageously selected to provide a cooling sensation. In yet other aspects, various combinations of the above stimulation materials 150 may be utilized.

Suitable polyols can be obtained from Roquette America, Inc., a company having offices in Keokuk, Iowa, U.S.A., under the trade name of XYLISORB (xylitol) or NEOSORB sorbitol. Such polyols can generally be obtained from the manufacturer in particular particle sizes, such as 90 microns, 300 microns, 500 microns, and 1000 microns and above.

Other suitable stimulation materials 150 that absorb heat during dissolution include salt hydrates, such as sodium acetate (H₂O), sodium carbonate (H₂O), sodium sulfate (H₂O), sodium thiosulfate (H₂O), and sodium phosphate (H₂O); anhydrous salts such as ammonium nitrate, potassium nitrate, ammonium chloride, potassium chloride, and sodium nitrate; organic compounds such as urea and the like or combinations thereof.

In some aspects, the stimulation material 150 can include a temperature change agent in the form of a warming agent, which can include those substances that release heat during dissolution from an insult. Examples of materials that release heat during dissolution include manganese chloride, aluminum chloride, aluminum sulfate, potassium aluminum sulfate, and the like or combinations thereof. In some aspects, the warming agent can also include those substances that release heat during swelling. The stimulation material 150 can also include ortho esters or ketals such as menthone ketals that result from reacting menthone with alcohols containing 1 to 8 carbons or polyols containing 2 to 8 carbons, and all structural and optical isomers thereof. Particular menthone ketals that may be suitable include menthone-glycerol ketal and menthone-propylene glycol ketal. Other suitable ketals are disclosed in U.S. Patent No. 5,348,750 to Greenberg and U.S. Patent No. 5,266,592 to Grub et al..

The selection of a particular temperature change agent and the determination of the amount to be used should be based in part on the desired temperature change. For example, in some aspects, the total amount of stimulation material 150 present in the stimulation composite 1 10 can range from a basis weight of from about 40 gsm to about 3000 gsm, such as about 100 gsm to about 2000 gsm, or about 300 gsm to about 1000 gsm.

In addition, as referenced above, in some aspects, the disposable absorbent article 20 (Figs. 9 and 10) desirably provides a surface temperature change when wet of from about 2 °C to about 20 °C. To achieve this result, the temperature change substance and the amount used should be selected so that the possible total energy change is from about 3 to about 30 calories per square centimeter (cal/cm²), which may represent either a possible total energy release of from about 3 to about 30 cal/cm² or a possible total energy absorption of from about 3 to about 30 cal/cm², such as from about 6 to about 24 cal/cm², or about 12 to about 18 cal/cm².

Temperature change agents that absorb or release heat on contact with an aqueous solution desirably have a heat of solution, hydration, or reaction greater than about 30 cal/g, or less than about -30 cal/g. The heat of solution, hydration, or reaction is suitably within the range of from about 30 to about 90 cal/g or from about -30 to about -90 cal/g, such as from about 30 to about 70 cal/g or from about -30 to about -70 cal/g, such as xylitol at -32 cal/g or urea at -60 cal/g.

In some circumstances, it may be desirable for the stimulation composite 110 to do more than just alert the user of an insult. Indeed, it may be desirable for the stimulation composite 110 to additionally provide a benefit to the user. For example, in addition to alerting the user of an insult, the stimulation device could also provide pH buffering for the skin, vaginal health-care for a female, odor control, coating materials for skin health, or even medicines.

Further, it may be desirable to use more than one stimulation material. For instance, a first stimulation material may cause a temperature change/sensation upon a first insult, and a second stimulation material may cause a temperature change/sensation upon a second insult due to the fact that the second stimulation material is in a different area. In the alternative, the two (or more) stimulation materials may be blended together. In one aspect, the first stimulation material has a temperature change upon a first liquid insult. A second stimulation material, having a need for more than one insult to for a physical or chemical reaction to occur (e.g. different solubility), may present a tingling sensation. Other combinations of stimulation material are within the scope of this invention.

In some aspects, a pledget of stimulation composite 110 can be adapted to provide the wearer with an expanding or contracting dimensional change sensation. Dimensional change elements of this type are described in more detail in U.S. Patent No. 5,649,914 to Glaug et al.. A pressure change agent in the form of a dimensional change material includes materials that rapidly undergo a change in at least one dimension when exposed to an aqueous solution. The dimensional change is suitably either as an expansion to at least about 2 times a dry dimension or as a contraction to less than about one-half (1/2) of the dry dimension. For example, the dimensional change material can have a wet height- dimension that is at least about 2 times greater than its dry height dimension, such as at least about 5 times greater for improved performance. The height dimension of the dimensional change material is in the z-direction 3 of the stimulation composite 1 10 so that the dimensional change is noticeable to the wearer of the absorbent article 20. In other aspects, the x-direction 1 and/or y-direction 2 of the stimulation composite 1 10 can additionally or alternatively remain the same, expand, or contract when exposed to an aqueous solution. Suitable materials for use in the dimensional change material include expandable foams, superabsorbents, or the like.

In some aspects, the pressure change agent produces a gas, such as carbon dioxide. Gas generating materials of this type are described in more detail in U.S. Patent No. 7,002,055 to Long et al.. The gas produced upon wetting with urine, complex fluids or other body exudates can produce a sound, a smell, a tingling sensation or apply pressure to the user.

In some aspects, the stimulation composite 110 can optionally include a surfactant. In some aspects, the gas producing materials can interact with the surfactant to produce a foam that applies pressure to the user. Thus, the surfactant component can be present as a foaming agent. For example, when a gas, such as carbon dioxide, is produced, the gas interacts with the surfactant and a bubble-filled foam is produced. These bubbles cause the article to swell and push against the skin of the wearer to alert the wearer of a liquid insult.

The surfactant used is not critical so long as it does not substantially irritate the skin upon contact, or adversely affect the efficacy of the stimulation material 150. A wide variety of surfactants may be suitable for use in accordance with the present invention. For example, suitable surfactants include anionic surfactants, nonionic surfactants, amphoteric surfactants, cationic surfactants, and combinations thereof. Examples of suitable anionic surfactants include alkyl benzene sulfonates, alkyl sulfates, alkyl ether sulfates, sulfosuccinates, and combinations thereof. Examples of suitable nonionic surfactants include ethoxylated alcohols, fatty acid alkanolamides, ethoxylated alkanolamides, amine oxides, and combinations thereof. Examples of suitable amphoteric surfactants include alkyl betaines, amidobetaines, and combinations thereof. Examples of suitable cationic surfactants include alkylammonium halides. In some aspects, the stimulation composite 110 can include from about 0.1 grams to about 15 grams of surfactant.

In other aspects, a pressure change agent in the form of a gas producing material can include an aqueous-soluble effervescent solid material. Such aqueous-soluble effervescent solid material typically comprises pressurized gas-containing cells. When the solid material having pressurized gas-containing cells is contacted with urine or other body exudates, the solid material begins to dissolve and the pressurized gas is released from the cells during dissolution of the solid material. In some aspects, this gas can interact with an optional surfactant and produce a foam and bubbles that cause the article 20 to press or apply pressure to the skin of the user.

In this aspect, the soluble effervescent solid material may include a sugar compound such as a mono-saccharide, di-saccharide, or poly-saccharide that has been infused with a gas that is substantially non-reactive with human skin. Suitable gases for infusion into a solid material include, for example, carbon dioxide, air, nitrogen, argon, helium, other substantially inert gases, and combinations thereof. Specific examples of saccharides that can be used in accordance with the present disclosure include glucose, fructose, sucrose, lactose, maltose, dextrin, cyclodextrin, and the like, or combinations thereof. Also, a mixture of sucrose with corn syrup (containing glucose, maltose, and dextrin) can be used in accordance with this aspect of the present disclosure to produce a gas-containing effervescent material. Other examples of compounds that are capable of being prepared in such a manner as to trap pressurized gas in cells include, for example, water soluble compounds such as salts, alkali halides, and alkaline earth metal halides. Specific salts useful in the present disclosure include, for example, sodium chloride, potassium chloride, potassium bromide, lithium chloride, cesium chloride, and the like. In some aspects, the cells containing the pressurized gas have a diameter of from about 5 micrometers to about 100 micrometers.

A substantially non-reactive gas can be infused into the cells of the soluble solid material to produce an effervescent material useful in the present invention by first heating the starting material, such as a sugar, in a small amount of water until the material is dissolved. After dissolution of the material, the water is evaporated off leaving the material in a molten state. The molten material is then gasified by introducing a suitable gas, such as carbon dioxide, at a superatmospheric pressure into a sealed vessel containing the molten material. The molten material is agitated during gasification to ensure intimate contact between the molten material and the gas. Pressures of, for example, between about 50 psig (340 kPa) and about 1000 psig (6890 kPa) may be utilized to infuse the gas into the molten material. After gas infusion, the molten material is allowed to solidify while maintained in the sealed vessel to produce an effervescent material. A suitable procedure of producing a gas- containing solid material is set forth in U.S. Patent No. 4,289,794 to Kleiner et al.. The above procedure can produce solid effervescent materials containing cells of pressurized gas from about 50 psig (340 kPa) to about 900 psig (6200 kPa) which, when exposed to urine, complex fluids or other body exudates, allow the release of the trapped gas.

In some aspects, the pressure change agent comprising a gas producing material includes at least one acid and at least one base. The acid and base react together upon being wetted to produce a gas that may be, for example, carbon dioxide gas. The exact gas produced by the gas producing system is not critical, so long as the gas produced is substantially non-harmful to the skin of the wearer.

In some aspects, the stimulation material 150 can be a gas-generating material that includes a polymeric acid and a complementary base, resulting in an acid-base reaction. Suitable acidic polymers should be readily water soluble or water-wettable. Examples of suitable polymeric acids include, but are not limited to, polyacrylic acids, polystyrene phosphorus acids, and the like. In some aspects, the polymeric acid can be a "bidentate" or higher order acid. By "bidentate or higher order" it is meant that the polymeric acid has more than one acid group in its smallest polymer building block. This can be easily understood when one compares ascorbic acid to tartronic acid (two acid groups) and citric acid (three acid groups). In some aspects, the polymeric acids may be a dendrimer or the like where the dendrimer's surface and interior are fully functionalized with acid groups.

Other examples of suitable acids include, but are not limited to, acetic acid, lactic acid, amino acid, ascorbic acid, glyolic acid, salicylic acid, tartaric acid, citric acid, EDTA, tartronic acid, polyacrylic acid, maleic acid, phosphonic acid, and the like. More materials such as these are referenced in U.S. Application Serial No. 12/646,763, filed on December 23, 2009.

### Adhesives

Materials that make suitable thermoplastic adhesives include those that exhibit adhesive properties when transitioning from a molten state to a solid state. In some aspects, the adhesive composition is suitably a pressure-sensitive hot-melt adhesive 152. Such an adhesive generally comprises one or more polymers to provide cohesive strength, such as aliphatic polyolefins such as polyethylene-co-propylene, polyamides, polyesters, and/or polyether blends; ethylene vinyl acetate copolymers; styrene-butadiene or styrene- isoprene block copolymers; and the like.

As an example, the pressure-sensitive hot-melt adhesive 152 may contain from about 15 to about 50 weight percent cohesive strength polymer or polymers; from about 30 to about 65 weight percent resin or other tackifier or tackifiers; from more than zero to about 30 weight percent plasticizer or other viscosity modifier; and optionally less than about 1 weight percent stabilizer or other additive. It should be understood that other hot- melt adhesive formulations comprising different weight percentages of these components are possible. It is also contemplated that the adhesive composition may either be hydrophilic or hydrophobic without departing from the scope of this invention.

Examples of suitable materials include hydrophobic and hydrophilic hot melt polymers, such as those available from National Starch and Chemical Co. (having a place of business located in Bridgewater, New Jersey, U.S.A.) such as 34-5610, 34-447A, 70- 3998 and 33-2058; those available from Bostik-Findley (having a place of business located in Milwaukee, Wisconsin, U.S.A.) such as HX 4207-01, HX 2773-01, H2525A, H2800, H9574; and those available from H.B. Fuller Adhesives (having a place of business located in Saint Paul, Minnesota, U.S.A.) such as HL8151-XZP. Other adhesives are further described in U.S. Patent Publication No. 2005/0096623 to Sawyer, et al. .

It is also contemplated that alternative adhesives may be used without departing from the scope of this invention. Examples of alternative adhesives include polyethylene oxide (PEO); polyethylene glycol (PEG); polyviny alcohol(PVOH); starch derivatives such as starch ethers, carboxymethyl starch, cationic starch, hydroxyalkyl starch, and the like, for example hydroxyethyl starch, hydroxypropyl starch and hydroxybutyl starch; cellulose derivatives such as cellulose ethers, hydroxyalkyl cellulose, for example hydroxyethyl cellulose, hydroxypropyl cellulose, methylcellulose, methyl propyl cellulose, carboxymethyl cellulose, and the like; polyacrylic acid; polyvinylmethyl ether; carrageenan; water-soluble alkyd resins; or the like, ethylene vinyl acetate copolymer (EVA) and combinations thereof. In addition, thermoplastic adhesive fibers, such as thermoplastic binder fibers, can also be used.

The basis weight of each thermoplastic meltblown adhesive 152 can range from about 1 gsm to about 150 gsm, such as about 20 gsm to about 100 gsm. In still other aspects, the total amount of thermoplastic adhesive in the thermoplastic meltblown adhesive 152 can be about 2-15 wt% of the total weight of all stimulation material in the stimulation blend 156.

### Substrates

Referring to Fig. 2, in one aspect, the stimulation composite 1 10 of the present invention is a laminate made with substrates 154 and 155, and a stimulation blend 156. The substrates 154 and 155 are materials that can be at least partially or completely liquid permeable. Suitable liquid permeable materials include tissue layers; nonwovens such as meltblown, coform, spunbond, spunbond-meltblown-spunbond (SMS), bonded-carded-web (BCW), woven fabric; perforated films; foam layers; HYDROKNIT (a nonwoven obtainable from Kimberly Clark Corporation having offices in Neenah, Wisconsin, U.S.A.), airlaid, cotton web and the like.

Each of the substrates 154 and 155 can suitably have a basis weight of about 10 gsm to about 100 gsm, such as about 10 gsm to about 80 gsm, or about 10 gsm to about 50 gsm. The substrates 154 and 155 may be identical or different materials.

### Physical Characteristics of the Stimulation Composite

Referring to Fig. 2 and 3, the stimulation composite 110 can have a cross-machine direction or "x-direction," and a machine-direction or "y-direction" which together can form a plane when in a laid-flat condition, hereinafter referred to as the "x-y plane." Referring to Fig. 3, the stimulation composite 110 also has a z-direction that is perpendicular to the x-y plane. The stimulation composite 110 has a body-facing surface 187 intended to be disposed toward the wearer in use (i.e., a body-facing surface), and a garment-facing surface 188 intended to be disposed away from the wearer in use.

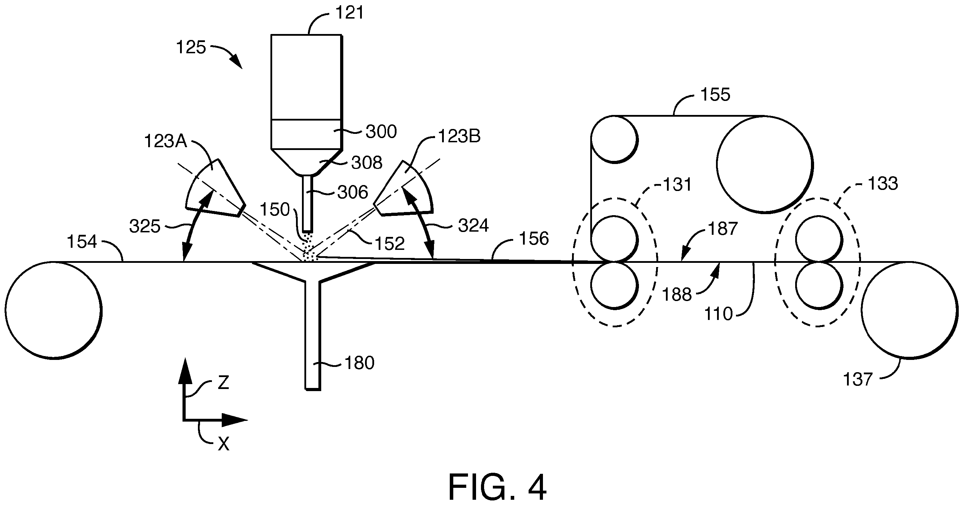

In one aspect, the liquid permeable network of meltblown pressure-sensitive hot-melt adhesive 152 used in the present invention (described supra) allows liquid to transfer though the stimulation composite 110 (when substrates 154 and 155 are also porous). In some aspects, the porosity of the substrates 154 and 155 and may be enhanced by piercing the stimulation composite 110 from the body-facing side 187 through the opposite garment-facing side 188, such as by a needle punch (see Fig. 4, optional aperture unit 133). Methods of perforating materials include, but are not limited to, needle-punch, air-jet, and the like.

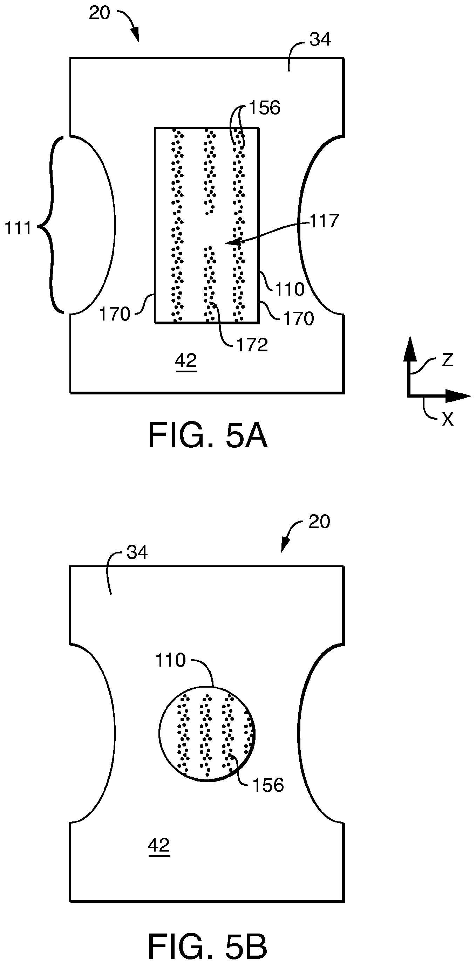

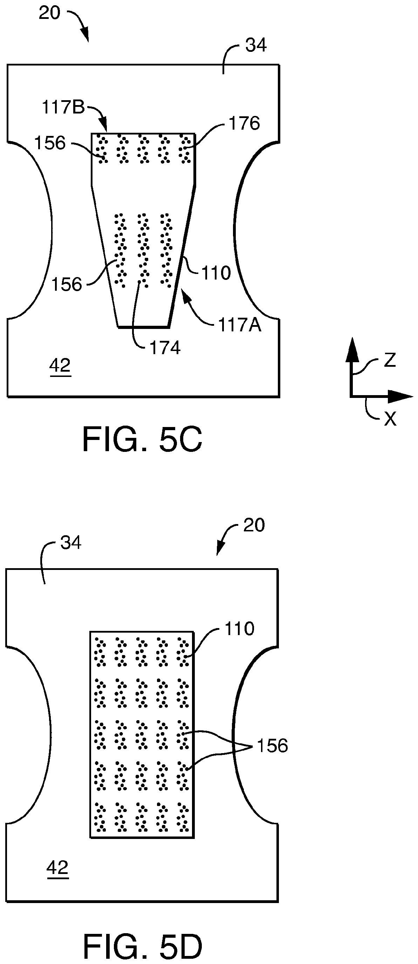

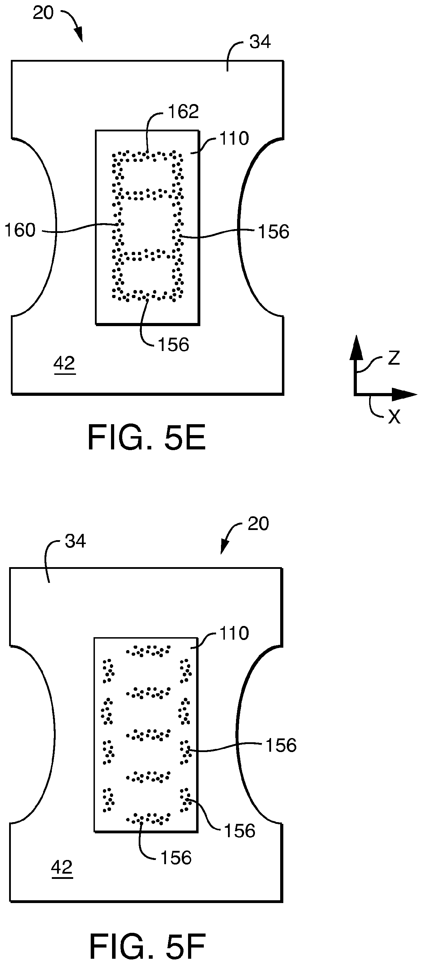

Referring to Figs. 5(A-F), the pledgets of stimulation composite 110 can have any desired shape. For example, it may be rectangular shaped, triangular shaped, circular shaped, oval shaped, race-track shaped, I-shaped, generally hourglass shaped, T-shaped and the like. In some aspects, the stimulation composite 110 may have no particular defined shape, but instead, have a random shape. Thus, the area in at least the x-y plane can vary as desired.

Referring to Figs. 1 and 3, the stimulation composite 110 also has a thickness 153 dimension in the z-direction. The thickness 153 of the stimulation composite 110 can vary due to the fact that while the surface area of stimulation composite 110 is coated with the meltblown pressure-sensitive adhesive 152, there are other sections further containing the stimulation materials as described herein. The sections having stimulation materials shown as peaks 113, and the sections containing only pressure-sensitive adhesive 152 are shown as valleys 115. Thus, the maximum thickness 153 of the stimulation composite 110 (as defined by the peaks 113) is between about 0.2 mm and 10 mm, such as between about 0.25 mm and 5 mm or between about 0.5 mm and 3 mm as measured by the Thickness Test. If there are secondary peaks 113, the thickness 153' of the secondary peaks 113 may be measured with a caliper.

### Patterns

There is an opportunity to make pre-determined functional patterns and/or aesthetic patterns defined by raised sections of stimulation blends 156, (should they be thick enough to be discernable). Either way, there is an opportunity to use pre-determined patterns of stimulant blend 156 such that the same stimulation effects can be obtained with less stimulation material 150. Further, patterns defining a predetermined insult pathway may be used to direct liquid insult(s) away from the target zone to achieve a sensation away from the target zone.

The surface area defined by the x-y plane of the body-facing side of a pledget of stimulation composite 110 includes a target zone where fluids are introduced upon insult. A first stimulation material 150 may be strategically located with respect to the target zone. A second stimulation material may be located in a second area with respect to the target zone and the first stimulation material. These separate areas provide an opportunity for delivering a sensation after not just one insult, but in addition, a second insult. Typically the first insult will define a first target zone, and the second insult will define a second target zone which is larger than the first target zone.

Referring to Figs. 5A-F, shown is only a small sampling of possible pledget shapes and patterns defined by the stimulation blend 156. In one aspect, Fig. 5A depicts an elongated pledget of stimulation composite 110 having a pair of stripes of stimulant blend 156 extending the length 111 of the pledget. These stripes are spaced apart in the x-direction and located in opposite sides of the pledget. In a central region 117 of the there are another pair of stripes of stimulation material 150 disposed along the longitudinal axis of the pledget. These stripes are spaced apart in the y direction. The resulting pledget is symmetric with respect to the x and y directions. In another aspect, Fig. 5B depicts the pledget of stimulation composite 110 in an oval shape. There are four spaced apart stripes of stimulation blend 156 extending the length (y-direction) of the pledget, and spaced apart in the x-direction. Desirably the stripes of stimulation blend 156 are symmetric in the x and y directions. In another aspect, Fig. 5 C depicts the pledget of stimulation composite 110 in an overall V-shape. There are two regions 117, a lower region 117A and an upper region 117B. The upper region 117B includes five stripes of stimulation blend 156, and the lower region 117A includes four stripes of stimulation blend 156. The pledget is symmetric about the y-axis (longitudinal axis). In another aspect, one can see that Fig. 5D depicts a rectangular-shaped pledget of stimulation composite 110 having an array of stripes of stimulation blend 156. The stripes of stimulation blend 156 are spaced apart in both the y-direction and the x-direction. In another aspect, one can see that Fig. 5E depicts a rectangular-shaped pledget of stimulation composite 110 having pattern of stimulation blend 156 wherein there are two longitudinal stripes of stimulation blend 156 spaced apart in the x-direction, and four stripes of stimulation blend 156 extending between the longitudinal stripes to from a ladder. Finally, FIG. 5F depicts another rectangular-shaped pledget of stimulation composite 110 having pattern of stimulation blend 156 wherein there are eight short stripes of stimulation blend 156, four on opposite sides of the pledget and spaced apart in both the x and y directions, and four transverse stripes of stimulation blend 156 spaced apart in the y-direction and extending from between the short stripes.

In other aspects (not shown), the patterns made by certain placement of stimulation blend 156 includes concentric rectangles, concentric ovals, concentric circles, wavy lines, cross pattern, stripes and combinations thereof. In one aspect, the overall pattern is symmetric with respect to a machine direction (y-direction) of the absorbent article. In yet another aspect, the pattern is symmetric with respect to a cross-machine direction (x-direction).

In another aspect as seen in Fig. 2, the stimulation blend 156 forms three stripes oriented in the y-direction, and spaced apart in the x-direction. The figure shows the material blend 156 in the cut-away section. This stripe has a width WA as measured in the x-direction. The middle stripe of material blend 156 has a width WB that is less than WA. It is at times desirable to vary the width of the stripes of material blend 156. In addition, as can be seen in Fig. 3, the height of an outer, exposed stripe of material blend 156 has a thickness 153, whereas the smaller middle stripe of material blend 156 has lesser thickness 153'.

It is noted that while most of the patterns formed with stimulation blend 156 are shown to be symmetrical in at least one direction in the x-y plane, it is not necessary to have a symmetrical design. The symmetry may be aesthetic or functional.

In one aspect, the stimulation blend 156 covers 10 to 90 percent of the surface area of the stimulation composite 110, or 20 to 60 percent of the surface area of the stimulation composite 110, or 40 to 50 percent of the surface area of the stimulation composite 110.

In another aspect, the stimulation blend 156 covers 20 to 100 percent of surface area of the stimulation composite 110, or 20 to 40 percent of the surface area of the stimulation composite 110.

In another aspect, the pressure-sensitive adhesive covers 95 to 100 percent of the surface area of the stimulation composite 110. Space between the areas containing stimulation blend 156 may be about 0 to 25 mm or about 5 to 15 mm or about 10 mm.

It is noted that regions of stimulation blend 156 are not sharply defined, and it is possible for insignificant amounts of stimulation material 150 to be located between the areas containing significant amount of stimulation blend 156. Relative to the stimulation blend 156, the stimulation material 150 fades to a small percentage in the areas of pressure-sensitive hot-melt adhesive 152. In one aspect, there may be about 0 to 10 percent of the stimulation material between the regions of stimulation blend 156.

It is noted that the adhesive 152 may not be significantly bound to any stimulation material 150. For example, the adhesive 152 located between stripes of stimulation blend 156 as seen in Figs. 2 and 3.

### Method of Making a Stimulation Composite

In general, construction of the stimulation composite 110 of the present invention can be accomplished by any suitable method well-known to those skilled in the art. In one aspect, a stimulation material 150 is partially bonded to the meltblown pressure-sensitive adhesive 152 to form a stimulation blend 156. The stimulation blend 156 can appear as raised sections if thick enough to be visually detected at the body-facing surface 187 (see Fig. 3) of the stimulation composite 110. After the manufacturing process shown in Fig. 4, the stimulation composite 110 is cut from the web into a pledget of a desired shape, some of which are described above with reference to Figs. 5A-F.

Fig. 4 illustrates one exemplary process for constructing the stimulation composite 110. A substrate 154 is supported on a support belt (not shown). The support belt is supported on two or more rolls (not shown) provided with suitable driving means (not shown) for moving the belt. The substrate 154 passes under a particulate feeder 121 having two adhesive meltblown applicators 123A and 123B flanking each side of the particulate feeder 121. Applicator 123A is located upstream of particulate feeder 121, and applicator 123B is located downstream of the particulate feeder 121.

Referring to Fig. 4, in one aspect, the adhesive applicators 123 A, B flank the particle feeder 121 at predetermined angles 324 and 325 within a range of about 35° to about 80°, or about 40° to about 60°, or about 45° to 50°. The predetermined angles need not be identical.

Desirably, the particulate feeder 121 is a modified CHRISTY Dry Material Dispensing Machine in which the unmodified portion is available from the Christy Machine Company of Freemont, Ohio, U.S.A. Together, the particulate feeder 121 and the adhesive applicators 123A, B define a deposit system 125.

In operation, the deposit system 125 serves to deposit stimulation material 150 (and/or any other beneficial additives) into an adhesive fiber stream, thereby forming a stimulation blend 156. Stimulation blend 156 may fully cover the substrate 154, or may cover only sections of substrate 154.

Desirably, there is a second substrate 155 applied to substrate 154. The stimulation composite 110 passes through a nip roll 131 and optionally, a needle punch 133 (should the permeability of substrates 154 and 155 not be high enough without the punch).

The resulting stimulation composite has three layers, a) a bottommost layer of substrate 154, which is desirably a liquid-permeable nonwoven material located on the garment-facing side of the absorbent article 20, b) a middle layer of comingled meltblown pressure-sensitive adhesive 152 and stimulation material 150, and c) the topmost layer of substrate 155, which is desirably a liquid-permeable nonwoven or other material located on the body-facing side of the absorbent article 20.

Desirably, the stimulation composite 110 has a ratio of meltblown pressure-sensitive adhesive 152 comingled with the stimulation materials 150 of about 15/85, or in another aspect about 10/90, or in yet another aspect about 5/95. Most desirably, the ratio of meltblown pressure-sensitive adhesive to stimulation materials is about 2/98.

The web of stimulation composite 110 may be rewound and eventually unwound for conversion into pledgets of stimulation composite 110, only a few of which are shown in Fig. 5 or discussed herein.

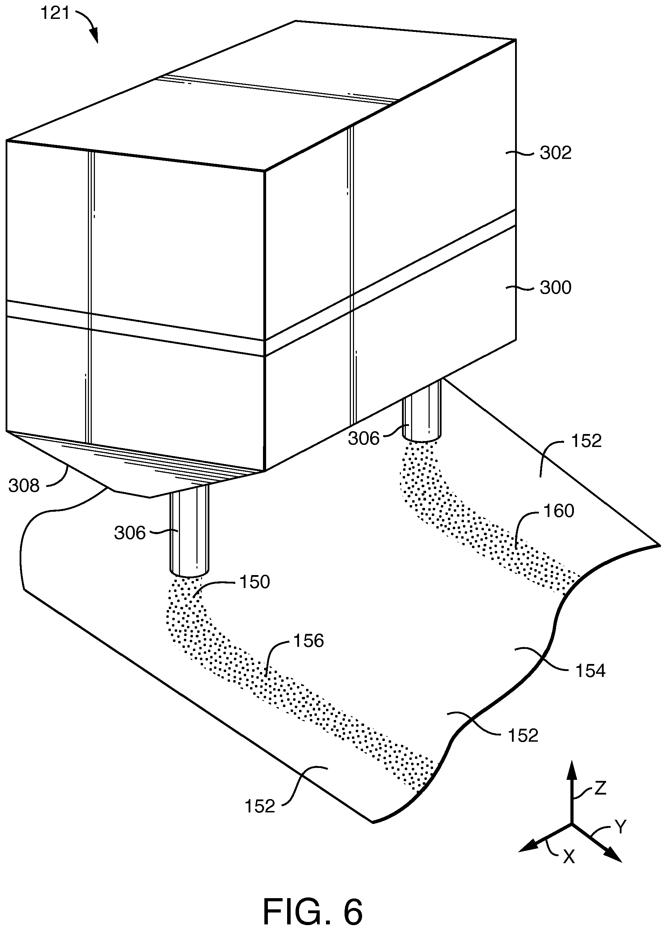

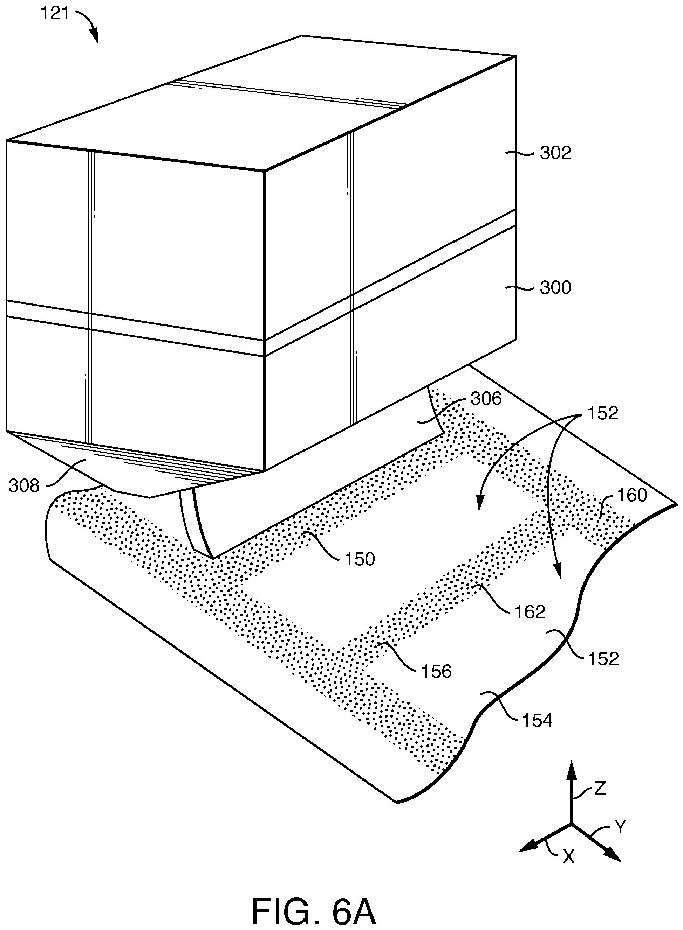

Referring to Figs. 4 and 6, a particle channel attachment 300 is connected to the outlet end 302 of the particle feeder 121 shown as a black box for ease of illustration. The particle channel attachment 300 has an array of channels 306 from which the stimulation material 150 flows. In a meltblown process, as the stimulation material 150 leaves each channel 306, it is immediately comingled with fibers of adhesive 152.

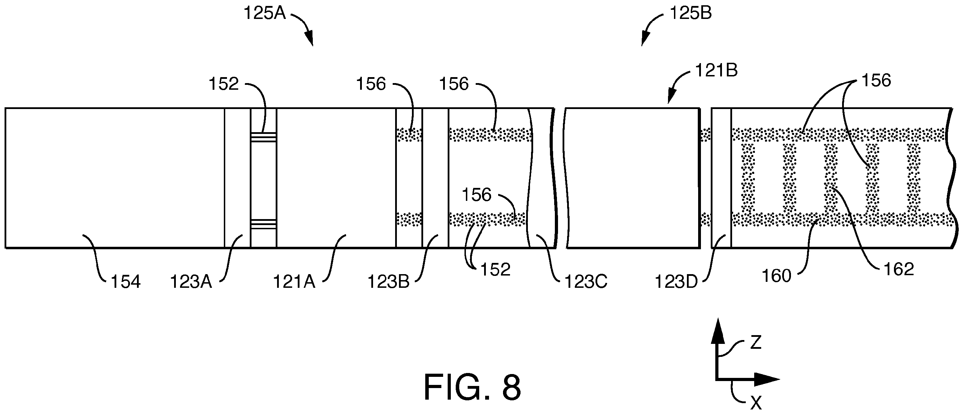

Referring now to Fig. 8, in one aspect, a stimulation composite 156 may be made by using more than one deposit system 125, referred to here as deposit systems 125A and 125B. In this embodiment, the deposit system 125A is the same as that previously described for Fig. 4. Likewise, the deposit system 125B is the same at that previously described for Fig. 4 with one exception, the particulate feeder 121B has different particle feeder attachments 300.

The particle channel attachment 300 may have channels 306 of the same shape and size as shown in Fig. 6, or they may differ in this respect. If each channel 306 is the same in size and shape, the stripes of stimulation blend 156 will be substantially uniform in dimension. If one of the channels 306 is a different size, such as smaller in width, the stripe of stimulation blend 156 will have smaller dimensions. One such result is shown in Fig. 3. Shown is variance in the amount of stimulation blend 156 and pressure-sensitive adhesive 152 deposited onto substrate 154.

Referring to the examples shown in Figs. 6, 6A and 8, the particle channel attachment 300 as used with deposit system 125A includes two channels 306 spaced apart in the x-direction. It may be seen how the resulting stripes of stimulation material 156 are oriented in the y-direction (machine direction). In Fig 6A, a single channel 306 is depicted. This single channel 306 extends in the x-direction (cross-machine direction). In this embodiment, the deposit system 125 B which uses the single channel 306 is pulsed so that the stimulation material 150 only flows periodically. The resulting web of stimulation composite 110 has continuous stripes 160 of stimulation blend 156 along each side of the substrate 154. In between the stripes 160 are transverse stripes 162. Together the stripes 160 and 162 form a ladder pattern.

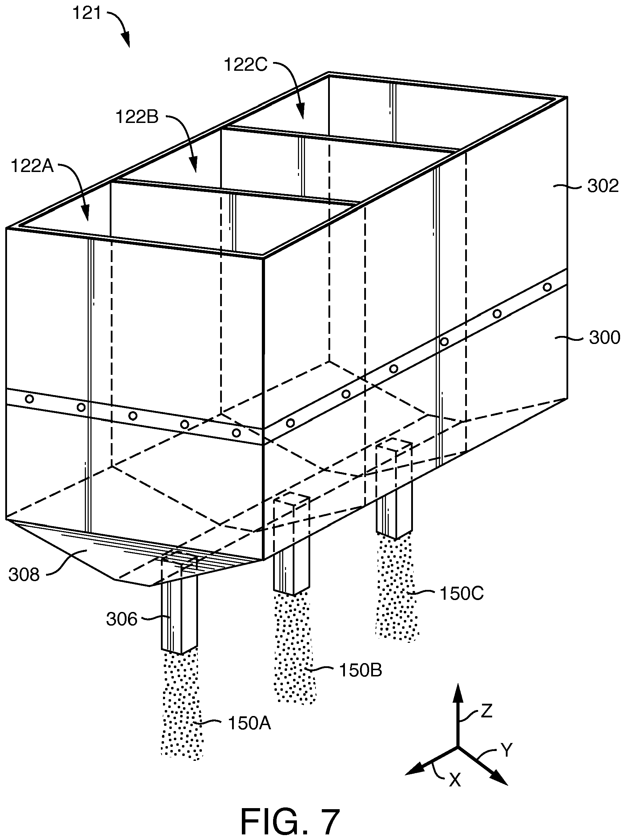

Referring to Fig. 7, in another embodiment, there are partitions 304 used to separate a single bin 122 (defined by the particulate feeder 121 with the particle channel attachment 300), into two or more bins such as 122A, B and C. Different particulate materials 150 can flow from each bin. For example, in bins 122A and 122C there may a particulate material that cools when exposed to liquid. In the middle bin 122b, there may be a particulate material that causes a tingling sensation to occur when exposed to liquid. Overall, any assortment of particulate materials may be placed into each bin 122 for a variety of stimulation effects felt by a wearer of an absorbent article in which the stimulation composite 110 is disposed.

Desirably, the particle channel attachment 300 (including channels 306) and panels 304 are made of a metal that does not react with the particulate matter passing through the particle feeder 121, e.g. stainless steel.

Referring back to Fig. 5A-F, one may now see how certain patterns of stimulation blend 156 are created by using differing arrays of channels 306 and/or multiple deposit systems 125. For example, Figs. 5A, C, E and F are made using two deposit systems 125. (Each deposit systems 125 may be used in a different order than described.) Specifically, Fig. 5A has a first deposit system 125 that lays down the outermost stripes 170. A second deposit system 125 lays down the central stripes 172 with a pulse so as not to apply a stimulant material 150 in the center of the stimulation composite 110. In Fig. 5C, a first deposit system 125 lays down three stripes 174, and a second deposit system 125 lays down five stripes 176 with a space between the sets of stripes. The pattern of Fig, 5E was previously described herein. Other patterns of stimulation blend 156 (such as those shown in Figs. 5B and 5D) may be made using only one deposit system 125. Overall, between the location of channels 306, the different sizes of channels 306, the ability to pulse the application of stimulation material 150 into the pressure-sensitive adhesive 152, and the use of more than one deposit system 125 allows one to make a wide variety of different patterns of stimulation blend 156. It is further contemplated that if the deposit system 125 had enough controls, it may be all that is needed to make all the designs shown in Fig. 5 (A-F) or even the more complex patterns.

### Exemplary Absorbent Articles

To gain a better understanding of the present invention, attention is directed to Figs. 9 and 10, shown is a training pant and a stimulation composite 1 10 of the present invention. It is understood that the present invention can also be suitable for use with various other disposable absorbent articles, including but not limited to the feminine pad of Fig. 1 1 or other personal care absorbent articles, health/medical absorbent articles, and the like, without departing from the scope of the present invention.

Various materials and methods for constructing training pants are disclosed in U.S. Patent No. 6,761,711 to Fletcher et al.; U.S. Patent No. 4,940,464 to Van Gompel et al.; U.S. Patent No. 5,766,389 to Brandon et al., and U.S. Patent No. 6,645,190 to Olson et al.

Fig.9 illustrates a training pant 20 in a partially fastened condition, and Fig.10 illustrates a training pant 20 in an opened and unfolded state. The training pant 20 defines a longitudinal direction 1 that extends from the front of the training pant when worn to the back of the training pant. Perpendicular to the longitudinal direction 1 is a lateral direction 2.

The training pant 20 defines a front region 22, a back region 24, and a crotch region 26 extending longitudinally between and interconnecting the front and back regions. The pant 20 also defines an inner surface (i.e., body-facing surface) adapted in use (e.g., positioned relative to the other components of the pant) to be disposed toward the wearer, and an outer surface (i.e., garment-facing surface) opposite the inner surface. The training pant 20 has a pair of laterally opposite side edges and a pair of longitudinally opposite waist edges.

The illustrated pant 20 may include a chassis 32, a pair of laterally opposite front side panels 34 extending laterally outward at the front region 22 and a pair of laterally opposite back side panels 734 extending laterally outward at the back region 24.

The chassis 32 includes a backsheet 40 and a topsheet 42 that may be joined to the backsheet 40 in a superimposed relation therewith by adhesives, ultrasonic bonds, thermal bonds or other conventional techniques. The chassis 32 may further include an absorbent core 44 such as shown in Fig. 10 disposed between the backsheet 40 and the topsheet 42 for absorbing fluid body exudates exuded by the wearer, and may further include a pair of containment flaps 46 secured to the topsheet 42 or the absorbent core 44 for inhibiting the lateral flow of body exudates.

The backsheet 40, the topsheet 42 and the absorbent core 44 may be made from many different materials known to those skilled in the art. All three layers, for instance, may be extensible and/or elastically extensible. Further, the stretch properties of each layer may vary in order to control the overall stretch properties of the product.

The backsheet 40, for instance, may be breathable and/or may be fluid impermeable. The backsheet 40 may be constructed of a single layer, multiple layers, laminates, spunbond fabrics, films, meltblown fabrics, elastic netting, microporous webs or bonded-carded-webs. The backsheet 40, for instance, can be a single layer of a fluid impermeable material, or alternatively can be a multi-layered laminate structure in which at least one of the layers is fluid impermeable.

The backsheet 40 can be biaxially extensible and optionally biaxially elastic. Elastic non-woven laminate webs that can be used as the backsheet 40 include a non-woven material joined to one or more gatherable non-woven webs or films. Stretch bonded laminates (SBL) and neck bonded laminates (NBL) are examples of elastomeric composites.

Examples of suitable nonwoven materials are spunbond-meltblown fabrics, spunbond-meltblown-spunbond fabrics, spunbond fabrics, or laminates of such fabrics with films, or other nonwoven webs. Elastomeric materials may include cast or blown films, meltblown fabrics or spunbond fabrics composed of polyethylene, polypropylene, or polyolefin elastomers, as well as combinations thereof. The elastomeric materials may include PEBAX elastomer (available from AtoFina Chemicals, Inc., a business having offices located in Philadelphia, Pennsylvania U.S.A.), HYTREL elastomeric polyester (available from Invista, a business having offices located in Wichita, Kansas U.S.A.), KRATON elastomer (available from Kraton Polymers, a business having offices located in Houston, Texas, U.S.A.), or strands of LYCRA elastomer (available from Invista), or the like, as well as combinations thereof. The backsheet 40 may include materials that have elastomeric properties through a mechanical process, printing process, heating process or chemical treatment. For example, such materials may be apertured, creped, neck-stretched, heat activated, embossed, and micro-strained, and may be in the form of films, webs, and laminates.

One example of a suitable material for a biaxially stretchable backsheet 40 is a breathable elastic film/nonwoven laminate, such as described in U.S. Patent No. 5,883,028, to Morman et al. Examples of materials having two-way stretchability and retractability are disclosed in U.S. Patent Nos. 5,1 16,662 to Morman and 5,1 14,781 to Morman. These two patents describe composite elastic materials capable of stretching in at least two directions.

The topsheet 42 is also sufficiently liquid permeable to permit liquid body exudates to readily penetrate through its thickness to the absorbent core 44. A suitable topsheet 42 may be manufactured from a wide selection of web materials, such as porous foams, reticulated foams, apertured plastic films, woven and non-woven webs, or a combination of any such materials. For example, the topsheet 42 may include a meltblown web, a spunbonded web, or a bonded-carded-web composed of natural fibers, synthetic fibers or combinations thereof. The topsheet 42 may be composed of a substantially hydrophobic material, and the hydrophobic material may optionally be treated with a surfactant or otherwise processed to impart a desired level of wetability and hydrophilicity.

The topsheet 42 may also be extensible and/or elastomerically extensible. Suitable elastomeric materials for construction of the topsheet 42 can include elastic strands, LYCRA elastics, cast or blown elastic films, nonwoven elastic webs, meltblown or spunbond elastomeric fibrous webs, as well as combinations thereof. Examples of suitable elastomeric materials include KRATON elastomers, HYTREL elastomers, ESTANE elastomeric polyurethanes (available from Noveon, a business having offices located in Cleveland, Ohio, U.S.A.), or PEBAX elastomers. The topsheet 42 can also be made from extensible materials such as those described in U.S. Patent No. 6,552,245 to Roessler et al. The topsheet 42 can also be made from biaxially stretchable materials as described in U.S. Patent No. 6,969,378 to Vukos et al.

The article 20 can optionally further include a surge management layer which may be located adjacent the absorbent core 44 and attached to various components in the article 20 such as the absorbent core 44 or the topsheet 42 by methods known in the art, such as by using an adhesive. In general, a surge management layer helps to quickly acquire and diffuse surges or gushes of liquid that may be rapidly introduced into the absorbent structure of the article. The surge management layer can temporarily store the liquid prior to releasing it into the storage or retention portions of the absorbent core 44. Examples of suitable surge management layers are described in U.S. Patent Nos. 5,486,166 to Bishop et al.; 5,490,846 to Ellis et al.; and 5,820,973 to Dodge et al.

The article 20 can further comprise an absorbent core 44. The absorbent core 44 may have any of a number of shapes. For example, it may have a 2-dimensional or 3-dimensional configuration, and may be rectangular shaped, triangular shaped, oval shaped, race-track shaped, I-shaped, generally hourglass shaped, T-shaped and the like. It is often suitable for the absorbent core 44 to be narrower in the crotch portion 26 than in the rear 24 or front 22 portion(s). The absorbent core 44 can be attached in an absorbent article, such as to the backsheet 40 and/or the topsheet 42 for example, by bonding means known in the art.

The absorbent core 44 can be formed using methods known in the art. While not being limited to the specific method of manufacture, the absorbent core can utilize forming drum systems, for example, see U.S. Patent No. 4,666,647 to Enloe et al., U.S. Patent No. 4,761,258 to Enloe, U.S. Patent No. 6,630,088 to Venturino et al., and U.S. Patent No. 6,330,735 to Hahn et al.

In some desirable aspects, the absorbent core includes cellulose fiber and/or synthetic fiber, such as meltblown fiber, for example. Thus, in some aspects, a meltblown process can be utilized, such as to form the absorbent core in a coform line. In some aspects, the absorbent core 44 can have a significant amount of stretchability. For example, the absorbent core 44 can comprise a matrix of fibers which includes an operative amount of elastomeric polymer fibers. Other methods known in the art can include attaching superabsorbent polymer particles to a stretchable film, utilizing a nonwoven substrate having cuts or slits in its structure, and the like.

The absorbent core 44 can additionally or alternatively include absorbent and/or superabsorbent material. Accordingly, the absorbent core 44 can comprise a quantity of superabsorbent material and optionally fluff contained within a matrix of fibers.

It should be understood that the absorbent core 44 is not restricted to use with superabsorbent material and optionally fluff. In some aspects, the absorbent core 44 may additionally include materials such as surfactants, ion exchange resin particles, moisturizers, emollients, perfumes, fluid modifiers, odor control additives, and the like, and combinations thereof. In addition, the absorbent core 44 can include foam.

While a training pant has been described above for exemplary purposes, it is understood that the stimulation blend of the present invention can be suitable for other personal care absorbent articles. For example, Fig. 1 1 shows an absorbent article 220 in the form of a feminine care pad having a topsheet 242, a backsheet 240, an absorbent core 244, a surge layer 246 and a peel strip 278. The absorbent article 220 further comprises a separate stimulation composite 110 disposed between the topsheet 242 and the surge layer 246.

The pledget of stimulation composite 110 as illustrated in the absorbent article figures is generally rectangular. However, as noted above, the pledget can have any of a number of shapes, including but not limited to those shown in Fig. 5.

In some aspects, the stimulation composite 110 can be attached in an absorbent article, such as to the absorbent core, a surge layer and/or a topsheet for example, by bonding means known in the art, such as ultrasonic, pressure, adhesive, aperturing, heat, sewing thread or strand, autogenous or self-adhering, or the like, and combinations thereof. In other aspects, the stimulation composite may be free-floating within the article.

Referring to Fig. 10, because the physical sensation resulting from the stimulation composite 110 is noticeable to the wearer, the wearer's ability to recognize when a liquid insult has occurred (and/or is occurring) will be enhanced. The stimulation composite 110 can be positioned within the article 20 in any operative location such that a user can detect a physical sensation as a result of the stimulation composite 110 receiving an aqueous liquid insult. For example, in some aspects, the stimulation composite 110 can be disposed adjacent to and in contact with the body-facing surface of an absorbent core 44. In other aspects, the stimulation composite 110 can be disposed adjacent to and in contact with the garment-facing surface and/or the body-facing surface of a topsheet 42. In still other exemplary aspects, the stimulation composite 110 can be disposed adjacent to and in contact with the body-facing surface or garment-facing surface of a surge layer, for example. Other configurations are also suitable for the invention as would be readily apparent to those skilled in the art.

In one embodiment, the pledget of stimulation composite 110 may be sized so that the pledget fits between the containment flaps 46. One reason for this is that the insulted portion of the diaper tends to sag, sometimes leaving the containment flaps 46 in contact with the skin. As such, one possible target zone could be the edges of a pledget on which there is contact with the pledget of stimulation composite 110. Further, it is contemplated that there could be separate stripes (not shown) of stimulation composite 110 disposed in regions of absorbent articles that remain in contact with the skin after an insult has occurred.

### Example

This example is a thermal imaging study of an absorbent article of the present invention (see Fig. 10). Per Table 1 below, various codes were tested, each having a sample size of 3. While the stimulation composite sample size is relatively small for each code, and each 8.4 cm x 10.2 cm (3.3 x 4 inch) sample of the stimulation composite had either three or four lanes of xylitol after being randomly cut from the larger eight-lane webs defined below in Table 1, one can still appreciate the differences in cooling between the control and the codes S, B and D.

**TABLE 1**

| **CODE** | **Stimulation Composite DESCRIPTION** | **BASIS WEIGHT (gsm)** | **AMOUNT OF XYLITOL PER 8.4 cm x 10.2 cm stimulation composite PATCH, (grams)** |
|---|---|---|---|
| C | Control-Uniform Xylitol | 750 | 6.0 |
| S | Pattern 2* | 424 | 3.4 |
| B | Pattern 3** | 465 | 3.7 |
| D | Pattern 3** | 580 | 4.6 |

| | | | |
|---|---|---|---|
| *Pattern 2 is formed by eight channels spaced 16mm apart and having a 12 by 16mm outlet. **Pattern 3 is formed by eight channels spaced 16mm apart and having a 13 by 10mm outlet. | | | |

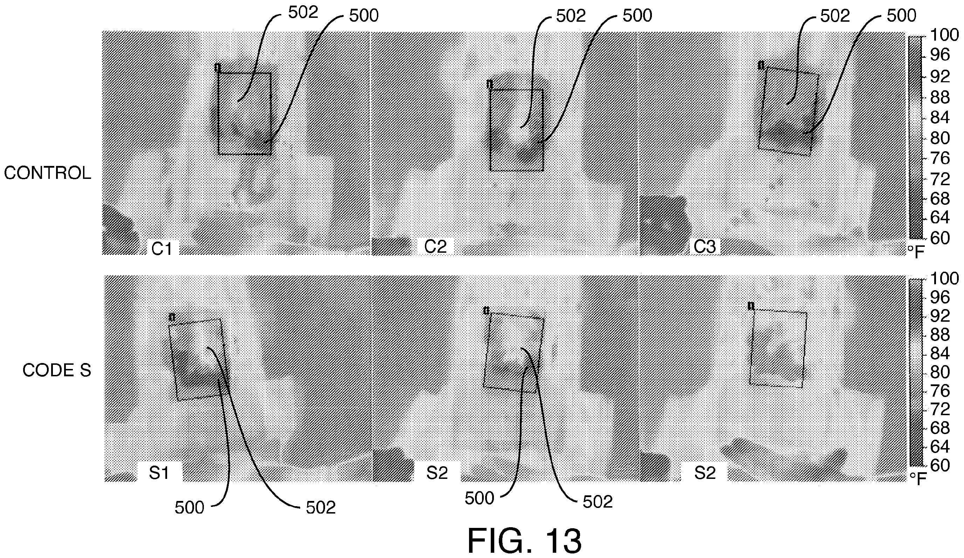

For example, Fig. 13 shows that there is a comparable cooling effectiveness of Code S with respect to the control, Code C. In each of the images, the darkest color 500 of blue represents about 68 degrees F. The yellow-orange section (shown just above the blue section) is where the insult occurred referred to as the target zone 502. The target zone is about 86 degrees F. With the exception of code S3 (which may have been damaged), one can see that the cooling effectiveness is about the same between the control and the sample containing about 45% less xylitol than the control.

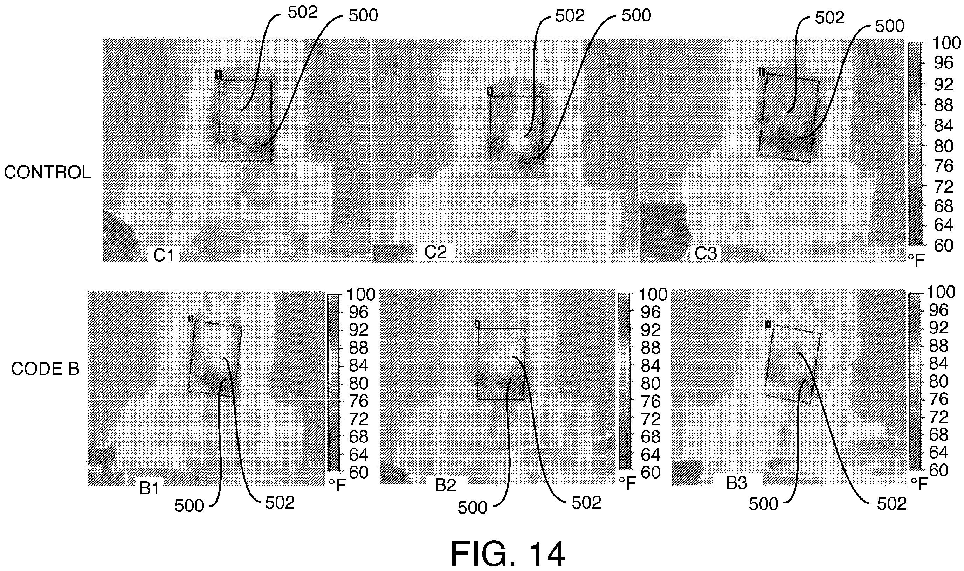

Fig. 14 shows that there is a similar cooling effectiveness of Code B with respect to the control, Code C. Once again, one can see that the cooling effectiveness is about the same between the control and the sample containing about 40% less xylitol than the control.

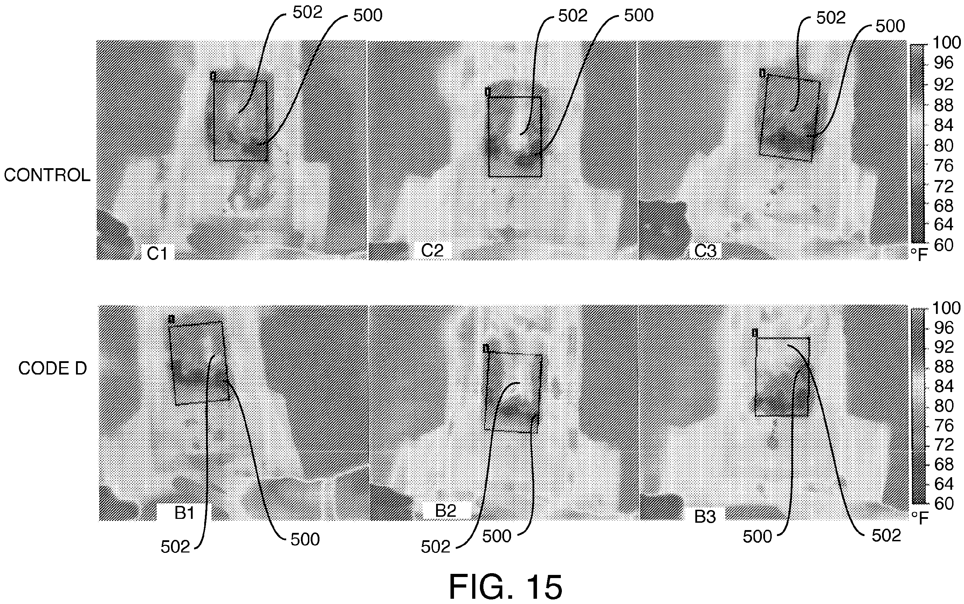

Fig. 15 shows that there is almost an equal cooling effectiveness of Code D with respect to the control, Code C. Once again, one can see that the cooling effectiveness is about the same between the control and the sample containing about 25% less xylitol than the control.

It can be concluded that all of the patterned samples (Codes S, B and D) are comparable to the control. The patterned stimulation composite are made with significantly less material, leading to a significant cost savings.

In addition to the cost savings, it can be seen that the xylitol is washed away from the yellow-orange target zone 502 where the insult was directed. This is a clue that the most efficient patterns of stimulation blend 156 might be those that do not have a significant amount of stimulation material in the pledget target zone. (Note that the region to which the insult is directed is likely to be gender specific.) It may be concluded that the stimulation material is best placed in the pledget regions that lie outside of the target zone 502 and which maintain skin contact after an insult has occurred. For instance, the blue areas 500 as seen in Figs. 13-15 may be the best pledget areas on which to place the stimulation blend 156.

### Test Methods

Unless otherwise noted, all tests are performed at a temp of 23 ±2 °C and a relative humidity of 50 ±5%.

### A. Stimulation Composite Thermal Imaging Test

For the test, samples of stimulation composite 110 were made with the following steps:
(1) Subjecting a spunbond-meltblown-spunbond (SMS) polypropylene carrier substrate 154 having a basis weight of 18 gsm (available from Kimberly-Clark Corporation, having a place of business in Neenah, Wisconsin, U.S.A.) to a deposit system 125. (Control samples are machine made and conveyed to the deposit system whereas the other samples are handmade.)
(2) At the deposit system 125, using a meltblown process at 160 °C, comingling the stimulation material 150 (xylitol) with fibers of adhesive 152 (made by the meltblown process) to create a stimulation blend 156. (The xylitol was a 700 FD xylitol obtained from Roquette America, Inc., a company having offices in Keokuk, Iowa, U.S.A.).
(3) The stimulation blend 156 and optionally, adhesive 152, was applied to a surface of substrate 154 in a striped pattern oriented in the y-direction (machine direction). A vacuum conveyor 180 (or just a vacuum source for hand-made samples) may be located directly underneath the deposit system 125 (see Fig. 4), to prevent most of the xylitol particles from spreading into region(s) of adhesive 152. The amount of stimulation material 150 within the stimulation blend 156 ranged from about 300 gsm to 750 gsm.
(4) Disposing a substrate 155 of 16 gsm tissue on top of the stimulation blend 156 and adhesive 152 to form a web of stimulation composite 110. The substrate 155 consisted of 100% NB 416, a bleached southern softwood Kraft pulp, available from Weyerhaeuser Co., having a place of business in Federal Way, Washington, U.S.A.
(5) The web of stimulation composite 110 was subjected to a 70 gram-force/square cm (50 psi) of pressure to the stimulation composite 110.
(6) To create the striped pattern of stimulation blend 156, an attachment 300 having eight particle channels 306 was attached to a particle feeder outlet end 302 (a CHRISTY brand particle feeder was used). The attachment 300 comingled eight separate streams of stimulation material 150 with the meltblown adhesive 152 fibers to form the stimulation blend 156. The resulting eight lanes of stimulation blend 156, separated by lanes of the adhesive 152, were oriented in the machine direction. These materials were applied to a surface of substrate 154.
(7) The stimulation composite 110 was cut into 8.38 cm x 10.16 cm (3.3 x 4 inch) pieces having either 3 or 4 lanes of stimulation blend then and placed into the absorbent article 20 as shown in Fig. 10, described supra. The difference between samples having 3 or 4 lanes of stimulation blend was negligible.

The test was a controlled, randomized, bench trial wherein saline was applied to the absorbent pant 20 (see below). The test objective was to see where stimulation material 150 migrated to due to fluid insult, and to determine the cooling effectiveness and temperature profile of each code.

In general, three samples per code of disposable, size 2, HUGGIES brand training pants were tested on a size 2 mannequin warmed to body temperature, 37 C (98.6 F). Likewise, a fluid insult of 0.9% saline was heated to body temperature, 37 C (98.6 F). Each training pant was loaded with 75mL of the 0.9% saline at a rate of 900ml/minute into each sample in the same target zone location.

At 30 seconds post insult, a Mikron 7200V infrared thermal imaging camera (obtained from Mikron Technology Inc. of Oakland, New Jersey, U.S.A.) was used to collect the thermal images seen in Figs. 13-15. These images allow one to visualize the temperature profile of each sample at that time.

### Procedure steps

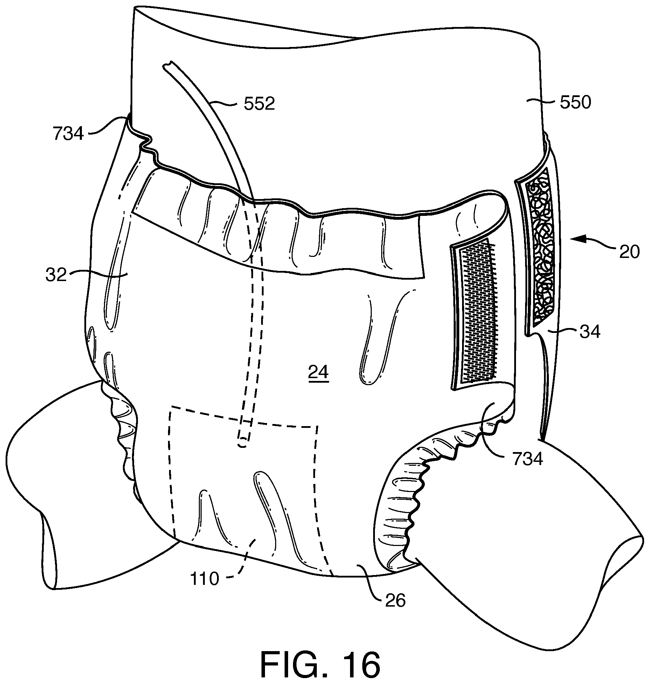

1. Set up thermal camera to collect 60 frames of data at a rate of 1 frame per second (fps) and an emissivity value of 0.98. The camera should be allowed a minimum of two (2) hours of warm-up time before testing begins. Non Uniformity Correction (NUC) should be completed every thirty (30) minutes throughout testing.
2. Place a mark on the liner of each of the products 100 mm from the top of the absorbent to designate the insult target.
3. Refer to Fig. 16. Prepare a mannequin 550 by running warm water in an over the mannequin until mannequin temperature reaches approximately 37.8 C (100 F).
4. Remove the mannequin 550 from the water and quickly dry the mannequin and apply the product to the mannequin per the randomization.
5. Place the mannequin 550 in a position on a surface 85 cm below the thermal imaging camera lens. The camera is focused on the surface of the mannequin 550.
6. Place pump tubing 552 down the front of the training pants 20 with the tube opening positioned at the marked target area.
7. Start thermal collection data followed immediately by the start of the insult. 75 ml of body temperature 0.9 percent saline is dispensed at a flow rate of 900 ml/min into the product.
8. Thirty (30) seconds after the insult completes, the training pants 20 are immediately removed from the mannequin 550 and placed flat on the surface allowing thermal imaging of the liner side of the pant to continue until all sixty (60) frames are captured.
9. Steps 3-8 are repeated for the remaining samples.

### B. Particle Size Test

A stack of sieves are used to determine the particle size distribution of a given sample. Thus, for example, a particle that is retained on a sieve with 710 micron openings is considered to have a particle size greater than 710 microns. A particle that passes through a sieve having 710 micron openings and is retained on a sieve having 500 micron openings is considered to have a particle size between 500 and 710 microns. Further, a particle that passes through a sieve having 500 micron openings is considered to have a particle size less than 500 microns, and so on.

The sieves are placed in order of the size of the openings with the largest openings on the top of the stack and the smallest openings on the bottom of the stack. Thus, all of the stimulation material associated with a stimulation blend can be weighed and placed into the sieve with the largest openings. Alternatively, if it is desired to determine the particle size or particle size distribution of the stimulation material in only a particular portion of the stimulation blend, only the stimulation material associated with that portion can be weighed and placed into the sieve with the largest openings. U.S. Standard sieves can be used in the sieve stack, including 20 mesh (850 microns), 25 mesh (710 microns), 35 mesh (500 microns), 50 mesh (300 microns) and 170 mesh (90 microns).

The sieve stack is shaken for 10 minutes with a Ro-Tap mechanical Sieve Shaker, Model RX29 available from W.S. Tyler of Mentor, Ohio, U.S.A. or other similar shaking device at standard test conditions. After shaking is complete, the stimulation material retained on each sieve is removed and the weight is measured and recorded. The percentage of particles retained on each sieve is calculated by dividing the weights of the particles retained on each sieve by the initial sample weight.

### C. Thickness Test

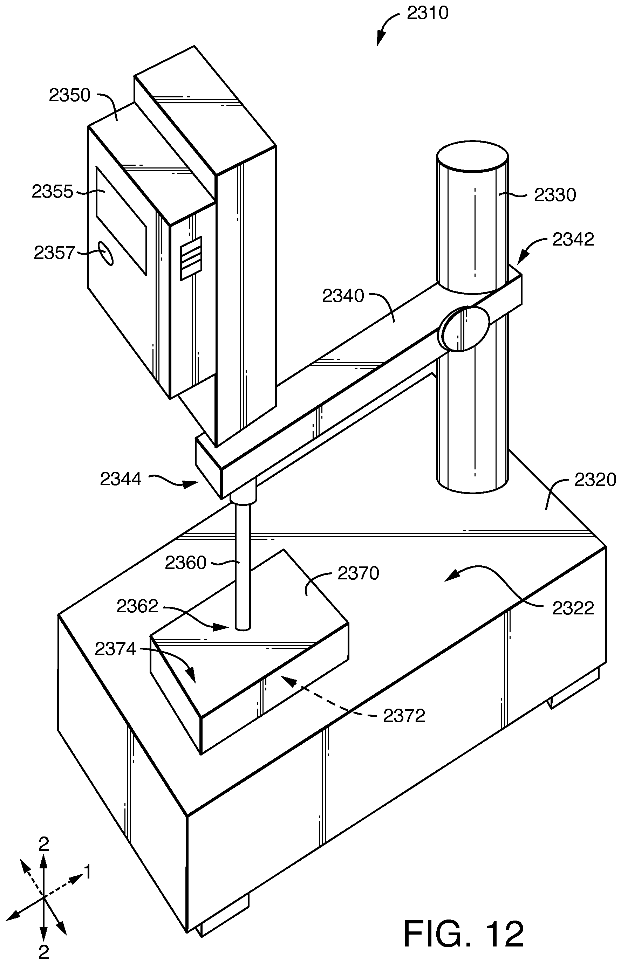

The thickness value of a selected portion or section of an article is determined using a thickness tester such as seen in Fig. 12. The thickness tester 2310 includes a granite base 2320 having a clamp shaft 2330 where the top surface 2322 of the granite base 2320 is flat and smooth. A suitable granite base is a Starret Granite Base, model 653G (available from The L.S. Starrett Company, having a place of business located in Athol, Massachusetts, U.S.A.) or equivalent. A clamp arm 2340 is secured to the clamp shaft 2330 at one end 2342 of the clamp arm 2340, and a digital indicator 2350 is secured to the clamp arm 2340 at the opposing end 2344. A suitable indicator is a Mitutoyo ID-H Series 543 Digimatic Indicator (available from Mitutoyo America Corp., having a place of business located in Aurora, Illinois, U.S.A.) or equivalent. Extending downward from the indicator 2350 is a vertically-movable plunger 2360.

To perform the procedure, a block 2370 having a length of 50 mm and a width of 44 mm is placed onto the granite base 2320. The block 2370 is constructed of acrylic and is flat and smooth on at least the bottom surface 2372. The thickness and weight of the block 2370 is configured such that the thickness tester 2310 provides a pressure to the sample of 0.69 kPa (0.1 psi). Next, the thickness tester 2310 is gently lowered such that the bottom surface 2362 of the plunger 2360 is in direct contact with the top surface 2374 of the block 2370 at the longitudinal 1 and transverse 2 center of the block 2370, and the plunger length is shortened by about 50%. The digital indicator 2350 is then tared (or zeroed) by pressing the "zero" button 2357. The digital display 2355 of the digital indicator 2350 should display "0.00 mm" or equivalent. The thickness tester 2310 is then raised and the block 2370 is removed. The test sample is then placed onto the top surface 2322 of the granite base 2320 and the block 2370 is gently placed on top of the test sample such that the block 2370 is substantially centered longitudinally 1 and transversely 2 on the sample. The thickness tester 2310 is then gently lowered again onto the block 2370 such that the bottom surface 2362 of the plunger 2360 is in direct contact with the top surface 2374 of the block 2370 at the longitudinal 1 and transverse 2 center of the block 2370, and the plunger length is shortened by about 50%, to provide a pressure of 0.69 kPa (0.1 psi). After 3 seconds, the measurement from the digital display 2355 is recorded to the nearest 0.01 mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, unless otherwise stated, a dimension disclosed as "40 mm" is intended to mean "about 40 mm."

While particular embodiments of the present invention have been illustrated and described, it would be obvious to those skilled in the art that various other changes and modifications can be made without departing from the scope of the invention as set out in the claims.

## Claims

1. A method of making a stimulation composite (110) comprising the following steps:
conveying a garment-facing substrate (155) to a deposit system comprising a particle feeder and a first adhesive applicator (123A) and a second adhesive applicator (123B) each flanking the particle feeder at predetermined angles, wherein the first adhesive applicator (123A) is located upstream of the particle feeder, and the second adhesive applicator (123B) is located downstream of the particle feeder;
emitting a stimulation material (150) from the particle feeder;
emitting pressure-sensitive hot-melt adhesive fibers (152) from the first and
second adhesive applicators to comingle the stimulation material (150) and adhesive fibers to create a stimulation blend (156); and
disposing the stimulation blend (156) onto the garment-facing substrate (155).

2. The method of claim 1 further comprising the steps of:
disposing a body-facing substrate (154) onto the stimulation blend (156); and
applying pressure to the stimulation composite (110).

3. The method of claim 1 or 2 wherein the predetermined angles range from 35 to 80 degrees.

4. The method of any preceding claim, wherein the stimulation blend (156) and the pressure-sensitive hot-melt adhesive fibers (152) are disposed onto the garment-facing substrate in a predetermined pattern.

5. The method of claim 4, wherein the predetermined pattern comprises alternating stripes of stimulation blend (156) and pressure-sensitive hot-melt adhesive fibers (152), wherein the stripes are oriented in a machine direction with respect to the stimulation composite.

6. The method of claim 4, wherein the pattern comprises alternating stripes of stimulation blend (156) and pressure-sensitive hot-melt adhesive fibers (152) wherein the stripes are oriented in a cross-machine direction with respect to the stimulation composite.

7. The method of claim 5 or 6 wherein the predetermined pattern comprises discontinuous stripes.

8. The method of any preceding claim, wherein said first and second adhesive applicators (123A, 123B) are adhesive meltblown applicators.

9. The method of any preceding claim, wherein the stimulation material (150) comprises a temperature change agent.

## Patentansprüche

1. Verfahren zur Herstellung eines Stimulationsverbundstoffs (110), umfassend die nachstehenden Schritte:
Transportieren eines zur Bekleidung weisenden Substrats (155) zu einem Abscheidungssystem, umfassend eine Teilchenzuführungsvorrichtung und ein erstes Klebstoffauftraggerät (123A) und ein zweites Klebstoffauftraggerät (123B), die jeweils die Teilchenzuführungsvorrichtung bei vorbestimmten Winkeln flankieren, wobei das erste Klebstoffauftraggerät (123A) stromaufwärts der Teilchenzuführungsvorrichtung angeordnet ist, und das zweite Klebstoffauftraggerät (123B) stromabwärts der Teilchenzuführungsvorrichtung angeordnet ist;
Ausgeben eines Stimulationsmaterials (150) aus der Teilchenzuführungsvorrichtung;
Ausgeben von Heißschmelzhaftklebstofffasern (152) von den ersten und zweiten Klebstoffauftraggeräten zum Vermengen des Stimulationsmaterials (150) und der Haftfasern miteinander, um ein Stimulationsgemisch (156) zu erzeugen; und
Abscheiden des Stimulationsgemischs (156) auf dem zur Bekleidung weisenden Substrat (155).

2. Verfahren nach Anspruch 1, weiter umfassend die Schritte von:
Anordnen eines zum Körper weisenden Substrats (154) auf dem Stimulationsgemisch (156); und Beaufschlagen von Druck auf den Stimulationsverbundstoff (110).

3. Verfahren nach Anspruch 1 oder 2, wobei die vorbestimmten Winkel im Bereich von 35 bis 80 Grad liegen.

4. Verfahren nach einem vorstehenden Anspruch, wobei das Stimulationsgemisch (156) und die Heißschmelzhaftklebstofffasern (152) auf dem zur Bekleidung weisenden Substrat in einem vorbestimmten Muster abgeschieden werden.

5. Verfahren nach Anspruch 4, wobei das vorbestimmte Muster abwechselnde Streifen von Stimulationsgemisch (156) und Heißschmelzhaftklebstofffasern (152) umfasst, wobei die Streifen in einer Maschinenrichtung hinsichtlich des Stimulationsverbundstoffs orientiert sind.

6. Verfahren nach Anspruch 4, wobei das Muster abwechselnde Streifen von Stimulationsgemisch (156) und Heißschmelzhaftklebstofffasern (152) umfasst, wobei die Streifen quer zur Maschinenrichtung hinsichtlich des Stimulationsverbundstoffs orientiert sind.

7. Verfahren nach Anspruch 5 oder 6, wobei das vorbestimmte Muster unterbrochene Streifen umfasst.

8. Verfahren nach einem vorstehenden Anspruch, wobei die ersten und zweiten Klebstoffauftraggeräte (123A, 123B) Geräte für Schmelzblas-Klebstoffauftrag sind.

9. Verfahren nach einem vorstehenden Anspruch, wobei das Stimulationsmaterial (150) ein Temperaturänderungsmittel umfasst.

## Revendications

1. Procédé de préparation d'un composite de stimulation (110) comprenant les étapes suivantes :
transporter un substrat faisant face à un vêtement (155) à un système de dépôt comprenant une alimentation de particules et un premier applicateur d'adhésif (123A) et un deuxième applicateur d'adhésif (123B) encadrant chacun l'alimentation de particules à des angles prédéterminés, dans lequel le premier applicateur d'adhésif (123A) est situé en amont de l'alimentation de particules, et le deuxième applicateur d'adhésif (123B) est situé en aval de l'alimentation de particules ;
émettre un matériau de stimulation (150) à partir de l'alimentation de particules ; émettre des fibres adhésives thermofusibles sensibles à la pression (152) à partir des premier et deuxième applicateurs d'adhésif pour mélanger le matériau de stimulation (150) et les fibres adhésives pour créer un mélange de stimulation (156) ; et
disposer le mélange de stimulation (156) sur le substrat faisant face au vêtement (155).

2. Procédé selon la revendication 1 comprenant en outre les étapes de :
disposer un substrat faisant face au corps (154) sur le mélange de stimulation (156) ; et
appliquer une pression au composite de stimulation (110).

3. Procédé selon la revendication 1 ou 2 dans lequel les angles prédéterminés varient de 35 à 80 degrés.

4. Procédé selon l'une quelconque des revendications précédentes, dans lequel le mélange de stimulation (156) et les fibres adhésives thermofusibles sensibles à la pression (152) sont disposés sur le substrat faisant face au vêtement dans un motif prédéterminé.

5. Procédé selon la revendication 4, dans lequel le motif prédéterminé comprend des bandes alternées du mélange de stimulation (156) et des fibres adhésives thermofusibles sensibles à la pression (152), dans lequel les bandes sont orientées dans une direction de la machine par rapport au composite de stimulation.

6. Procédé selon la revendication 4, dans lequel le motif comprend des bandes alternées du mélange de stimulation (156) et des fibres adhésives thermofusibles sensibles à la pression (152), dans lequel les fibres sont orientées dans une direction transversale à la machine par rapport au composite de stimulation.

7. Procédé selon la revendication 5 ou 6 dans lequel le motif prédéterminé comprend des bandes discontinues.

8. Procédé selon l'une quelconque des revendications précédentes, dans lequel lesdits premier et deuxième applicateurs d'adhésif (123A, 123B) sont des applicateurs par fusion soufflage d'adhésif.

9. Procédé selon l'une quelconque des revendications précédentes, dans lequel le matériau de stimulation (150) comprend un agent de changement de température.
